(19) Europäisches Patentamt
European Patent Office
Office européen des brevets

(11) Publication number: 0 256 748
A2

(12) **EUROPEAN PATENT APPLICATION**

(21) Application number: 87306887.8

(22) Date of filing: 04.08.87

(51) Int. Cl.⁴: B29C 55/00 , //B29K27:18

(30) Priority: 04.08.86 US 892271

(43) Date of publication of application:
24.02.88 Bulletin 88/08

(84) Designated Contracting States:
DE FR GB IT

(71) Applicant: BAXTER TRAVENOL LABORATORIES, INC.
One Baxter Parkway
Deerfield, IL 60015(US)

(72) Inventor: Tu, Roger H.
24755 Scott Lane
Lake Forest California 92630(US)
Inventor: Chen, David T.
18790 Teton Circle
Fountain Valley California 92708(US)
Inventor: Roberts, Mark A.
425 Merrimac, Apt. D302
Costa Mesa California 92626(US)

(74) Representative: Tubby, David George et al
MARKS & CLERK 57-60 Lincoln's Inn Fields
London WC2A 3LS(GB)

# (54) Porous highly expanded fluoropolymers and a process for preparing them.

(57) An interpenetrated polymeric matrix of poly(tetrafluoroethylene) and an elastomer is formed into a composite material which is durable and highly porous. The composite material can be used to produce shaped articles such as films, tubes, rods, and filaments with excellent pliability and elasticity. More particularly, the composite material can be utilized to produce vascular grafts with excellent biological compatibility.

EP 0 256 748 A2

## POROUS HIGHLY EXPANDED FLUOROPOLYMERS AND A PROCESS FOR PREPARING THEM

The present invention relates to a composition comprising a tetrafluoroethylene polymer and an elastomer and which is particularly suitable for surgical uses.

The chemical inertness and desirable physical properties of tetrafluoroethylene polymers and, in particular, of poly(tetrafluoroethylene) have caused them to become extremely popular and utilized in various manufactured articles.

US A-2 586 357 describes a paste-forming process of dispersing polymerized poly(tetrafluoroethylene) in a hydrocarbon lubricant and is well known commercially. Extrusions of various cross sectional shapes such as tubes, rods and tapes are commonly obtained from a variety of tetrafluoroethylene resins, and other paste-forming operations such as calendering and moulding are practiced commercially. The steps in paste-forming processes include mixing the resin with a lubricant, such as odourless mineral spirits, and carrying out forming steps in which the resin is subjected to compaction, thus making the shaped articles cohesive. The lubricant is removed from the extruded shape, usually by drying. In usual practice, this unsintered product is heated above the polymer's melting point, generally about 327°C, causing it to sinter or coalesce to an essentially impermeable structure.

JA B-13560/67 describes a process for producing a porous structure comprising tetrafluoroethylene. A mixture of uncured tetrafluoroethylene resin and a liquid lubricant is moulded into a sheet, rod, tube or strip by extrusion, rolling or other combination, and then heated at a temperature of about 327°C or above in an unsintered state, after which it is drawn in at least one direction. The structure is then drawn again in at least one direction at about the same temperature. The structure produced by this process has a specific gravity of 0.5 and a porosity as high as about 75%.

US A-3 953 566 describes a process for producing porous expanded paste-formed products of tetrafluoroethylene polymer having high tensile strength. Unsintered shapes of the polymer can be expanded by stretching in one or more directions within a preferred temperature range of 35°C to 327°C. Preferred resins utilized in this process have a high degree of crystallinity, preferably in the range of 98% or above, and a correspondingly low amorphous content. Small amounts of monomers, about 0.2% by weight, can be added to the poly(tetrafluoroethylene), and examples include ethylene, chlorotrifluoroethylene or fluorinated propylenes.

US A-4 187 390 describes products made from the process described in US A-3 953 566, including tubes which can be utilized as replacements for human arteries and veins.

While conventional processes can be used to produce the product of this invention, this invention relates to a product and process where there is an improvement in the porosity, flexibility and elasticity of the products produced therefrom. This invention relates to the rods, tubes, sheets, or any other shaped articles produced from tetrafluoroethylene, but more particularly relates to medical implants produced from porous poly(tetrafluoroethylene). In this invention, the emphasis is directed to the formation of medical implants or specifically, vascular grafts as shaped articles of poly(tetrafluoroethylene) and is not intended to limit the application of the novel process and other products described herein.

Conventional vascular grafts manufactured from porous poly(tetrafluoroethylene) have limitations in their strength and compliance. The porous grafts do not hold sutures or resist dilatation unless wrapped with a reinforcing film for support. This reinforcement slows down the tissue ingrowth and prevents rapid healing. This is because of the relatively low tensile strength or low tearing strength of the poly(tetrafluoroethylene). In addition, the grafts are stiff and noncompliant compared with a natural artery. A porous structure that is closer compared with a natural vessel would help prevent the complications resulting from the aforementioned characteristics.

The present invention thus provides a composite material comprising unsintered poly(tetrafluoroethylene) polymer and an elastomer which is expanded and oriented and having a porous fibrillated structure.

Shaped articles, including medical implants can be produced from poly(tetrafluoroethylene) and an elastomer preferably selected from the group consisting of polyvinylidene fluoride co-hexafluoropropylene, poly[tetrafluoroethylene-co-perfluoro(methyl vinyl ether)], poly(tetrafluoroethylene-co-propylene), poly(vinylidene co-chlorotrifluoroethylene), silicones, fluorosilicones, fluoroalkoxy phosphazenes, segmented copolyester ether, styrene-butadiene block copolymers, polyethers, acrylonitrile butadienes, isoprenes and mixtures thereof.

FIGURES 1 and 2 are schematic representations of the process of this invention;

FIGURES 3 through 9c are photomicrographs of products of this invention as shown in the Examples.

Porous poly(tetrafluoroethylene) vascular grafts are fabricated by a conventional process of paste extrusion followed by heating and stretching. The above noted patents describe processes which have been utilized in the manufacture of shaped articles of poly(tetrafluoroethylene). Minor amounts of other monomers and copolymers can be added to the processes wherein they function as fillers and lubricants to the poly-(tetrafluoroethylene). Conventional processes for producing poly(tetrafluoroethylene) can be utilized in the practice of this invention. Medical implants made from the composite material from the process of this invention exhibit excellent pliability, elasticity, durability and improved biological compatibility. Implants made from the composite material of this invention undergo endothialization rapidly. The composite material, when formed into a vascular graft and inserted in vitro, enmeshes into the tissue of the vessel so that smooth, thin cells penetrate into the graft, thus securing it and facilitating blood flow.

In this invention, it has been found that conventionally formed composite materials that are paste formed, dried, unsintered shapes which are expanded by stretching in one or more directions under certain conditions are suitable for the manufacture of medical implants. The process includes the addition of an elastomer to the poly(tetrafluoroethylene) resin prior to forming. The elastomer is preferably selected from the group consisting of polyvinylidene fluoride co-hexafluoropropylene, poly[tetrafluoroethylene-co-perfluoro-(methyl vinyl ether)], poly(tetrafluoroethylene-co-propylene), poly(vinylidene-co-chlorotrifluoroethylene), silicones, fluorosilicones, fluoroalkoxy phosphazenes, segmented copolyester ether, styrene-butadiene block copolymers, polyethers, acrylonitrile butadienes, isoprenes and mixtures thereof. The elastomer may be added to the poly(tetrafluoroethylene) in amounts effective to produce the desired results in a medical implant. Preferably, these amounts range from about 2% by weight to about 50% by weight of the poly-(tetrafluoroethylene). More preferably, the amount of elastomer that should be added to poly-(tetrafluoroethylene) to produce the desired results of this invention is about 10% by weight of the poly-(tetrafluoroethylene).

While any of the aforementioned elastomers function in this invention, a copolymer of propylene and tetrafluoroethylene [i.e. poly(tetrafluoroethylene)-co-propylene], sold under the tradename Aflas and manufactured by Asahi Glass Company is preferred. It has a structure in which tetrafluoroethylene and propylene arrange alternately in an orderly manner as shown:

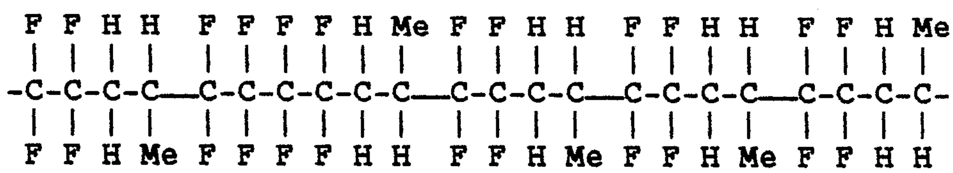

(Me = methyl).

Another preferred elastomer is silicone.

The porous structure obtained by the process of this invention is composed of fine fibres of poly-(tetrafluoroethylene) possibly coated with the elastomer. The elastomer encapsulates and reinforces the poly(tetrafluoroethylene) fibrils. The elastomer is a continuous matrix interpenetrating the microstructure of the fibrils. It modifies the sharp edges of the fibrils and nodes to render the edges smooth. The smooth edges of the fibrils and nodes create a poly(tetrafluoroethylene) elastomer product resistant to suture tearing with high intrinsic elasticity. The structure generally has a specific gravity ranging from about 0.2 to about 1.3, preferably about 0.6, and a porosity ranging from about 40% to about 90% void. The porosity allows for tissue ingrowth without allowing red blood cells to pass through the walls of the graft. The porous structure is opaque, but permeable to gases. The grafts are biologically compatible in that they function within normal biological actions and are not rejected. The elastomer present in the graft absorbs the kinetic energy of pulsating blood, thus providing durability to the graft that is missing in conventional grafts made from poly(tetrafluoroethylene).

The matrix tensile strength of the porous structure of this invention is in the range of 6000 - 7000 p.s.i. (422 - 492 kg/cm$^2$). In Example 3 which follows, a method for calculating matrix tensile strength is described. While the matrix tensile strength appears to be relatively high, the structure is strong but pliable and elastic. The structure, unlike prior art devices, is not stiff and possesses the characteristics (pliability, durability and biological compatibility) needed to enable it to function as a medical implant.

As stated, conventional processes can be utilized to produce the porous expanded paste-formed products of this invention. Typically, processes such as described in US A-3 953 566, may be utilized in conjunction with the addition of an elastomer as described herein to produce a composite material that can be formed into shaped articles having excellent pliability, elasticity and durability. Any conventional process for obtaining an unsintered moulding by a paste extrusion process, the calendering process or a combination of them may be utilized with the addition of an elastomer to produce the unexpected results described herein.

Accordingly, the polymeric matrix of this invention may be obtained by combining dispersions or emulsions of poly(tetrafluoroethylene) and the elastomer and coagulating the blend. Dispersions of poly-(tetrafluoroethylene) preferably contain from 10 to 50% by weight poly(tetrafluoroethylene) in water. More preferably, about 25% by weight is utilized. The elastomer dispersion preferably contains from 10 to 50% by weight of the elastomer. More preferably about 25% by weight of the elastomer dispersion is utilized to provide a mixture with the poly(tetrafluoroethylene) dispersion so that the elastomer is present in an amount equal to about 10% by weight of poly(tetrafluoroethylene) in the blended dispersions. The amounts of each of the poly(tetrafluoroethylene) and elastomer dispersions, to form the blended dispersion, may be varied so that the elastomer may be present in the polymetric matrix in amounts ranging from e.g. about 2% by weight to about 50% by weight (of the shaped article).

After coagulation, preferably with acetone, the coagulate may be filtered, washed, e.g. with ethanol, and dried. At this point it is preferably mixed with a lubricant. Alternatively, at this point, the process of producing the polymeric matrix may begin if one desires to use poly(tetrafluoroethylene) powder. Poly-(tetrafluoroethylene) in dry powder form, which is commercially available, may be admixed with a dispersion or emulsion of an elastomer. For example, the elastomer may be dispersed in solvent and sprayed on the poly(tetrafluoroethylene) powder while the powder is tumbling. The spraying should be accomplished on an intermittant basis to avoid lumping. Regardless of the source of the poly(tetrafluoroethylene), the blended poly(tetrafluoroethylene)-elastomer-lubricant mixture is left to stand to allow for lubricant dispersion.

The powder is then moulded, as by extrusion, rolling or a combination thereof. For a paste extrusion process, the powder is compressed to form a preform or billet which is extruded under conditions of elevated temperature and pressure.

The cut extrudate is preferably heated to a temperature between 35°C and its crystalline melting point, 327°C, and expanded. Typically, the temperature may be below the melting point, e.g. about 300°C. Expansion of the extrudate may be accomplished biaxially or uniaxially. With reference to uniaxial expansion, the nodes are elongated, the longer axis of a node being oriented perpendicular to the direction of expansion. The fibrils are oriented parallel to the direction of expansion. The rate of stretch may vary and is not intended to be limiting. Preferably the rate of stretch is 10% per second, however, it may range from 10% per second to about 100% per second. Another preferred rate of stretch is 30% per second. A suitable poly(tetrafluoroethylene)-elastomer resin matrix can be stretched and retain its desired functionality when heated to 35°C to 327°C, preferably 300°C. When heated at these temperatures, the poly-(tetrafluoroethylene) in the matrix has a high degree of crystallinity, preferably in the range of 98% or above, and a correspondingly low amorphous content. Conventional teachings suggest that annealing the poly(tetrafluoroethylene) resin at high temperatures just below the melt point improves the resin in the expansion process. The resin-elastomer matrix has expansion characteristics similar to poly-(tetrafluoroethylene)but, however, produces an improved product.

After expansion, the matrix is preferably sintered by insertion into an oven at a temperature ranging from 342°C to 390°C for a short period of time.

With respect to an alternative step, the sintered matrix may be immersed in an elastomer dispersion containing from 1 to 10% by weight elastomer. This step helps to rejoin any unconnnected elastomer components in the matrix. Additionally, poly(tetrafluoroethylene) products produced by conventional processes, such as the process described in US A-3 953 566, may be immersed in the elastomer dispersion containing 1 to 10% by weight elastomer to gain the benefits of the improvements shown by this invention. Conventionally produced poly(tetrafluoroethylene) products may absorb up to about 10% by weight elastomer from the elastomer dispersion. As a result, their physical properties, i.e. pliability, elasticity, etc., are improved, and they become suitable for use as compliant medical implants.

As is evident from the foregoing, the elastomer may be added to the poly(tetrafluoroethylene) by any number of methods within the knowledge of those skilled in the art.

As illustrated in Figures 1 and 2, a typical process for producing an interpenetrated polymeric matrix of poly(tetrafluoroethylene) ( = PTFE) and an elastomer is described as follows:

Step 1--Blending: Aqueous dispersions of poly(tetrafluoroethylene), and a fluoroelastomer are blended with very mild stirring. About 25% by weight of an elastomer, such as Aflas, is dispersed in water. About 25% by weight poly(tetrafluoroethylene) is dispersed in water and the solutions are combined.

Step 2--Coagulating: The blended dispersion is then coagulated by adding acetone while agitating vigorously. The coagulum floats to the top.

Step 3--Filtering: The coagulum is vacuum filtered to remove as much water and acetone as possible.

Step 4--Washing: The filtered coagulum is then washed repeatedly with ethanol and water to extract the surfactant which was in the suspension.

Step 5--Drying: After washing the coagulum, now a powder, it is dried to volatize any entrapped water or ethanol.

Step 6--Blending/Compounding: The PTFE powder is combined with about 10 to 30% by weight of a hydrocarbon or similar organic compound to serve as a lubricant. Suitable such compounds are kerosene, mineral spirits, alcohol, glycol and aromatics. The blended powder must stand several hours for uniform lubricant dispersion.

Step 7--Preforming: The lubricated powder is placed into a cylinder with a core rod in the center. The powder is then compressed to 300 to 500 p.s.i. (21 to 35 kg/cm$^2$) which forms a solid form, called a preform or billet.

Step 8--Paste Extrusion: The preform is placed in an extruder which, under hydraulic pressure, forces the material out of the die. The extrudate material is then cut into sections of predetermined lengths.

Step 9--Expansion: The extrudates are dried to evaporate the lubricant. As a result of the additon of the elastomer, the expansion parameters, such as temperature, rate and ratio, can be augmented with equally good results. Generally, the extruded shapes, e.g. tubes, are heated within a temperature range of from 35°C to 327°C, preferably to about 300°C, which is below the crystalline melting point. Alternatively, the matrix may be formed into a sheet or film. The preferred shape, however, is a tube that is utilized as a vascular graft.

Step 10--Flash sintering: Once expanded into tubes, they are inserted into a preheated oven at a temperature ranging from 342°C to 390°C for a relatively short period of time.

Step 11--Elasticity Recovering: Optionally the sintered poly(tetrafluoroethylene) tubes are immersed in an elastomer soluble solution containing about 5% by weight elastomer to form a continuous elastomer phase within the poly(tetrafluoroethylene) matrix.

The addition of elastomers to the poly(tetrafluoroethylene) improves the physical properties of medical implants and specifically, vascular grafts made therefrom. Among these properties are handling and suture retention, elasticity, pliability, durability, and biological compatibility.

The following examples describe the processes and products within this invention as well as a further description of the properties of the expanded tetrafluoroethylene polymers/elastomers. As indicated above, some of the properties of these expanded products are substantially different from the corresponding properties of conventional extruded or moulded tetrafluoroethylene polymers. As a result of these differences, the expanded composite material is useful in many applications involving medical implants and vascular grafts.

EXAMPLE 1

JSR Aflas elastomer, a copolymer of propylene and tetrafluoroethylene manufactured by Asahi Glass Company, was dissolved in ethyl acetate and diluted with Freon TF (trade mark), manufactured by DuPont, in about a 10% by weight solution. The diluted elastomer solution was then mixed with poly-(tetrafluoroethylene) powder while tumbling. The poly(tetrafluoroethylene) powder is sold under the trade name CD123 and manufactured by ICI Americas.

About 20 percent mineral spirits lubricant, on the final solid basis, was added to the poly-(tetrafluoroethylene)-elastomer mixture. The mixture was exposed to air for solvent evaporation. The final lubricant concentration was controlled at about 12% by weight. The lubricated powder was placed into a cylinder with a core rod in the center. The powder was then compressed to 300 to 500 p.s.i. (21 to 35 kg/cm$^2$) which formed a solid form called a preform or billet. The preform was placed in an extruder which under hydraulic pressure forces the material out of the die. The extruded material was cut into sections of a predetermined length.

The extruded material having average dimensions of 4 inch (~10 cm) length and 6 mm inside diameter were loaded onto a rack in an expansion oven. The oven temperature was then raised to 175°F (79°C) and held for ten minutes. The extrudates were thereafter expanded to 20 inches (~51 cm) at an expansion rate of 9.6% per second. The expanding grafts were removed from the oven and loaded on a separate rack for sintering and postcuring. The empty oven was heated to 650°F (343°C) and held for five minutes. The oven was then opened and the graft loaded rack brackets placed into the oven. The oven was closed, and the oven temperature raised to 670°F (354°C) and held for one minute for sintering. The sintered grafts were finally postcured at 450°F (232°C) for 16 hours in the oven.

EXAMPLE 2

A mineral spirit lubricant was added to the Fluon CD-123 poly(tetrafluoroethylene) powder while the powder was tumbling. No elastomer was added. Otherwise the procedure was as in Example 1. The well lubricated poly(tetrafluoroethylene) powders were compressed in a preformer to form a billet. The billet was extruded in accordance with the procedure of Example 1. A low expansion rate of 10% per second at the expansion temperature of 175°F (79°C), and above were utilized. The extrudates slipped out of the ties and were not expanded.

EXAMPLE 3

TENSILE STRENGTH

The tensile strength of a material is a minimum tensile stress, expressed in force per unit cross sectional area of the specimen, which the specimen will withstand without breaking. For porous materials, the cross sectional area of solid polymer within the polymeric matrix is not the cross sectional area of the porous specimen, but is equivalent to the cross sectional area of the porous specimen multiplied by the fraction of solid polymer within that cross section. This fraction of solid polymer within that cross section is equivalent to the ratio of the specific gravity of the porous specimen itself divided by the specific gravity of the solid polymeric material which makes up the force matrix. Thus, for example:

$\sigma$ = Standard tensile strength in p.s.i.

$P_1$ = Density of solid polymer, g/cm$^3$

$P_2$ = Density of expanded polymer, g/cm$^3$

$$\sigma_m = \frac{(\sigma)\,(P_1)}{(P_2)} = \text{Matrix tensile strength}$$

$\sigma$ = 2500 p.s.i., $P_1$ = 2.2, (for solid PTFE) $P_2$ = 0.40 (for expanded PTFE)

$$\sigma_m = \frac{(2500)\,(2.2)}{(0.40)} = 13{,}750 \text{ p.s.i. } (\sim 967 \text{ kg/cm}^2)$$

Matrix tensile strengths of grafts comprising various blends of polytetrafluoroethylene Aflas elastomer are shown in the following table:

| PTFE Wt. % | Aflas Elastomer Wt. % | Matrix tensile Strength, p.s.i. ($kg/cm^2$) |
|---|---|---|
| 90% | 10% | 6880 (484) |
| 70% | 30% | 4285 (301) |

EXAMPLE 4

SUTURE RETENTION TEST

The addition of elastomers of polytetrafluoroethylene improves the physical properties of the polytetrafluoroethylene vascular grafts. Among them, handling and suture retention are major factors. A test was devised to measure that suture retention and it is described as follows: (1) equipment used for testing: a) Chatillon digital dynamometer, b) Ethicon 6-0 prolene suture with C-1 taper needle and c) a pair of haemostats. (2) A hook attachment was assembled to the end of the dynamometer. (3) A suture was run through a sample of about 2 mm from the edge of the sample and at a 90° angle. A loop was made with the suture. (4) The looped end was placed with haemostats over the hook attachment on the dynamometer at a zero reading. (5) The sample was held with the left hand and the right hand secured the scale. The test sample was pulled away from the hook attachment at a rate of 5 inches (~13 cm) per minute. The pulling was continued until the suture had torn through the sample. (6) A reading was taken when the suture broke through the sample. This reading was the suture retention strength of that sample. Accordingly, this procedure was performed on various vascular grafts and the results are as follows.

Bentley A = 630 grams
Bentley B = 544 grams
Impra-I Vascular Graft = 214 grams
Gore-tex without outer sheath = 308 grams
Gore-Tex with outer sheath = 407 grams

Bentley sample A having a suture retention of 630 grams was produced from a mixture of poly(tetrafluoroethylene) and the elastomer, Aflas copolymer manufactured by the Asahi Glass Company, at a level of 30% by weight. The Bentley sample B was made from an Aflas elastomer and poly(tetrafluoroethylene) mixture wherein the amount of Aflas in the mixture was 10% by weight. Figure 3 shows the lumen surface of Bentley A in a microphotograph from scanning electron microscopy which clearly indicates typical poly(tetrafluoroethylene) fibril-nodal structures. This confirms that a 70% poly(tetrafluoroethylene) 30% Aflas vascular graft comprises primarily poly(tetrafluoroethylene) microstructures and Aflas to provide desired elasticiity. Figure 3a shows the scanning electron microscopy cross section of the Bentley A graft. Bentley A, the poly(tetrafluoroethylene)-Aflas graft, shows better elastic property than the 100% poly(tetrafluoroethylene)-10% Aflas grafts, Bentley B. Figures 3b and c show the lumen surface and cross section of Bentley B. The Gore vascular grafts, as seen in Figures 3d and e, show the lumen surface and cross section of the Gore-Tex graft. Figures 3d and e indicate typical fibrils-nodal microstructure. However, the cross section of both samples A and B indicates irregular fibrils comprised of poly(tetrafluoroethylene) and elastomer. The elastomer coated fibrils improve the physical properties of poly(tetrafluoroethylene) grafts help to close the suture holes at the anastomoses.

EXAMPLE 5

Silastic (trade mark) Q7-2213 is a medical grade dispersion manufactured by Dow Corning. It is a dimethylsiloxane elastomer dispersed in 1,1,1-trichloroethane. A dispersion of Silastic Q7-2213, containing 13% solids content, was diluted with 1,1,1-trichloroethane. The solution was mixed with 382.5 grams of Fluon CD-123 poly(tetrafluoroethylene) from ICI Americas to form a 15% silicone-85% poly(tetrafluoroethylene) powder mixture on a solid basis. Adequate mineral spirits were added to the mixture

while tumbling. After removing the solvent, 1,1,1-trichloroethane, the lubricated powder was preformed and extruded to make 6 mm inside diameter extrudates. Five extrudates were then heated to 320°F (160°C) for two hours in an effort to evaporate the lubricant. Subsequently, the extrudates were heated to 620°F (327°C) for thirty minutes and expanded. The expanded poly(tetrafluoroethylene)-silicone tubes were sintered at 661°F (349°C) for two minutes.

Figure 4 shows the lumen surface of this tube using scanning electron microscopy. It clearly demonstrates a typical fibril-nodal microstructure as that in an expanded poly(tetrafluoroethylene)-silicone tube in Figure 4a appears to have silicone-coated poly(tetrafluoroethylene) fibrils and nodes. The addition of silicone renders the poly(tetrafluoroethylene)-silicone tube more pliable and softer than a poly-(tetrafluoroethylene) tube.

EXAMPLE 6

A 6 mm vascular graft produced in accordance with the procedure of Example 5 was sterilized in ethylene oxide and then used to replace a portion of a canine aorta. The vascular graft was attached to the aortic artery proximally via interrupted suture technique. The other end was clamped so that the blood inside the graft was quasi-stationary for 30 minutes. This was done in order to study the blood-material interaction and the effect of pulsatile blood pressures between systolic and diastolic pressures. No blood seepage was observed. Figure 5 shows the expanded vascular graft. The whole lumen surface was clean and thrombus free which indicates excellent blood and material compatability. The little thrombus around the suture lining in the clamp site is a result of non-smoothness of haemodynamic flow. This demonstrates that poly(tetrafluoroethylene)-silicone is an excellent material for vascular grafts.

EXAMPLE 7

The poly(tetrafluoroethylene)-Aflas vascular grafts produced in Example 1 were sterilized with ethylene oxide. The grafts were inserted into the aorta of five canines so that a study of their efficacy could be performed. The grafts were patent and functioning well at the time of sacrifice: One canine at one month, one canine at two months, and two canines at three months.

Figure 6 shows the explant specimen for one of the canines after three months implant. The flow surface is generally clean and smooth with a uniform pseudointmal layer. Typical healing patterns were shown. Figure 6a is a lower power cross sectional microscopic view from midportion of a three month graft from another canine. A sinlge celled layer of endothelialization was observed. The tissue ingrowth was uniform throughout the graft cross section.

EXAMPLE 8

Extrudates made in accordance with the procedure of Example 1 were cut to 2.86 inches (7.26cm) in length and loaded onto a rack in an expansion oven. Procedures in accordance with Example 1 were followed. However, the expansion and sintering steps were modified to produce a 600% expanded tube containing 90% poly(tetrafluoroethylene) and 10% Aflas. The expanded tubes were soaked in Freon TF solvent for 10 minutes and air dried thereafter. Control tubes were soaked in a Freon TF Solution containing 2% by weight Aflas for ten minutes followed by air drying.

Compliance is defined as the percent change of graft volume per unit pressure increase. The control expanded tube, as shown in Figure 7, exhibited little compliance. The control expanded tube shows typical fibral-nodal structures as described in Example 4. The Freon TF treated tube is shown in Figure 7a. The exterior surface of fibrils and nodes becomes smoother because a very thin layer of Aflas elastomer was spread over them. The Freon TF treated tube shows substantially improved compliance because the elastomer component was rejoined together as a continuously interpentrated phase.

Figure 7b shows the lumen surface of the tube dipped in a 2% Aflas solution. More Aflas elastomer was added into the lumen surface to form a protective layer onto all the fibrils and nodes. Compliance was excellent.

EXAMPLE 9

Three commercially available Gore-Tex poly(tetrafluoroethylene) vascular grafts were obtained and were subjected to immersion procedures similar to those shown in Example 8 to generate improved elasticity. The grafts were labeled a, b and c respectively. Graft a was 17.6 cm long. It was tied onto a mandril at 14.196 cm. This corresponds to a 15% retraction. Graft a on the mandril was immersed in a Freon TF solution containing 1% Aflas elastomer for 30 minutes. It was then soaked in Freon TF for 3 minutes and air dried. Graft a showed a weight gain of 1.8% and minimal elasticity. The outside suface of the control Gore-Tex sheath, Graft d, is shown in Figure 8a while the outside surface of the once-soaked Graft a is shown in Figure 8b.

Graft b was tied into a mandrill at 15% retraction prior to immersion/soaking, similar to the procedure used for the Graft a. However, Graft b was soaked twice in a procedure similar to that used with Graft a. The weight gain of the Aflas elastomer was 4.1%. The graft exhibited substantial elasticity. Figure 8c shows a scanning electron microscopy lumen surface, while the outer surface without the outer sheath is shown in Figure 8d. The fibril nodal microstructure was coated with Aflas elastomer to enhance the elasticity on the graft.

EXAMPLE 10

Three tubes of poly(tetrafluoroethylene) with 5% Aflas elastomer were produced in accordance with the procedure of Example 1 and Figure 2. The tubes were labeled a, b and c, respectively. They were soaked in a Freon-TF solution containing 1% Aflas elastomer and were restricted to 0%, 15% and 25% retraction, respectively. Figures 9 a through c show Grafts a through c, respectively. Graft a, with 0% retraction looks very similar to nontreated poly(tetrafluoroethylene) Aflas elastomer grafts, as seen in Figure 3b, in its node and fibril structure. The fibrils begin to stick together in Graft b, showing the 15% retraction. The fibrils start to form thick bonds or the possible bonding of several fibrils by the Aflas elastomer. Graft c, with the 25% retraction, shows almost complete bonding of all fibrils. The surface appears to have lost most of its porosity with the fibrils being folded. Graft b with the 15% retraction appears to have the desired porosity.

The formation of the composite material by this invention, which can be shaped into medical implants or vascular grafts, can be accomplished by using poly(tetrafluoroethylene) and elastomers under conventional processing. The elastomers preferably comprise from about 2 to about 50% by weight of the shaped article and are expanded.

## Claims

1. A composite material comprising unsintered poly(tetrafluoroethylene) polymer and an elastomer which is expanded and oriented and having a porous fibrillated structure.

2. A composite material in accordance with Claim 1 in which the elastomer is polyvinylidene fluoride co-hexafluoropropylene, poly[tetrafluoroethylene-co-perfluoro(methyl vinyl ether)], poly(tetrafluoroethylene-co-propylene, poly(vinylidene-co-chlorotrifluoroethylene), a silicone, a fluorosilicone, a fluoroalkoxyphosphazene, a segmented copolyester ether, a styrene-butadiene block copolymer, a polyether, an acrylonitrile butadiene copolymer, an isoprene polymer or a mixture of any two or more thereof.

3. A composite material according to Claim 1 or Claim 2, which exhibits biological compatibility.

4. A composite material according to any one of the preceding Claims, which has an elastomer content of from 2 to 50% by weight of the composite material.

5. A composite material according to any one of the preceding Claims, which is expanded biaxially.

6. A composite material according to any one of Claims 1 to 4, which is expanded uniaxially.

7. A composite material according to any one of the preceding Claims, which has been stretched and then heated to a temperature ranging from 342°C to 390°C.

8. A composite material according to any one of the preceding Claims, wherein the elastomer is poly-(tetrafluoroethylene-co-propylene).

9. A composite material according to any one of Claims 1 to 7, wherein the elastomer is a silicone.

10. A shaped article formed from a composite material according to any one of the preceding Claims.

11. A shaped article according to Claim 10, which is in the form of a surgical prosthesis.

12. A shaped article according to Claim 10 or Claim 11, which is in the form of a rod.

13. A shaped article according to Claim 10 or Claim 11, which is in the form of a tube.

14. A shaped article according to Claim 10 or Claim 11, which is in the form of a film.

15. A shaped article according to Claim 10 or Claim 11, which is in the form of a vascular graft.

16. A process for the production of a porous article, which process comprises expanding an unsintered shaped article according to any one of Claims 10 to 15 comprising highly crystalline poly(tetrafluoroethylene) and an elastomer made by a paste-forming extrusion technique, by stretching said unsintered shaped article and maintaining said shaped article at a temperature between 35°C and the crystalline melting point of the tetrafluoroethylene polymer during said stretching.

17. A process according to Claim 16, in which the rate of stretch is about 10% per second.

18. A process according to Claim 16, in which the rate of stretch is about 30% per second.

19. A process according to Claim 16, in which the rate of stretch is about 100% per second.

20. A process according to Claim 16, in which said expanding is effected uniaxially.

21. A process according to Claim 16, in which siad expanding is effected biaxially.

22. A process according to any one of Claims 16 to 21, in which said expanding produces a porosity of from 40 to 90% in the resultant porous stretched article.

23. A process according to any one of Claims 16 to 22, wherein the shaped article is heated to a temperature ranging from about 342°C to about 390°C after stretching.

24. A process according to any one of Claims 16 to 23, wherein the shaped article is submerged in an elastomer dispersion.

25. A process according to Claim 24, wherein where the elastomer dispersion contains from 1 to 10% elastomer.

26. A process according to any one of Claims 16 to 25, wherein the elastomer is poly(tetrafluoroethylene-co-propylene).

27. A process according to any one of Claims 16 to 25, wherein the elastomer is a silicone.

28. A process according to any one of Claims 16 to 23, wherein the elastomer which is added to the poly(tetrafluoroethylene) to form a paste is in the form of a dry powder.

29. A process according to any one of Claims 16 to 23, wherein the elastomer which is added to the poly(tetrafluoroethylene) to form a paste is in the form of a liquid dispersion.

30. A process for the production of a porous article of a polymer of poly(tetrafluoroethylene) which process comprises expanding a shaped article comprising highly crystalline poly(tetrafluoroethylene) made by a paste-forming extrusion technique, by stretching said unsintered shaped article and maintaining said shaped article at a temperature between 35°C and the crystalline melting point of the tetrafluoroethylene polymer during said stretching, wherein the shaped article is submerged in an elastomer dispersion.

31. A process according to Claim 30 wherein the elastomer dispersion comprises from 1 to 10% elastomer.

32. A process according to Claim 30 wherein the elastomer is polyvinylidene fluoride co-hexafluoropropylene, poly[tetrafluoroethylene-co-perfluoro (methyl vinyl ether)], poly(tetrafluoroethylene-co-propylene), poly(vinylidene-co-chlorotrifluroethylene), a silicone, a fluorosilicone, a fluoroalkoxyphosphazene, a segmented copolyester ether, a styrene-butadiene block copolymer, a polyether, an acrylonitrile butadiene, an isoprene polymer or a mixture of any two or more thereof.

33. A process according to Claim 32 wherein the elastomer is poly(tetrafluoroethylene-co-propylene).

34. A process according to Claim 32 wherein the elastomer is a silicone.

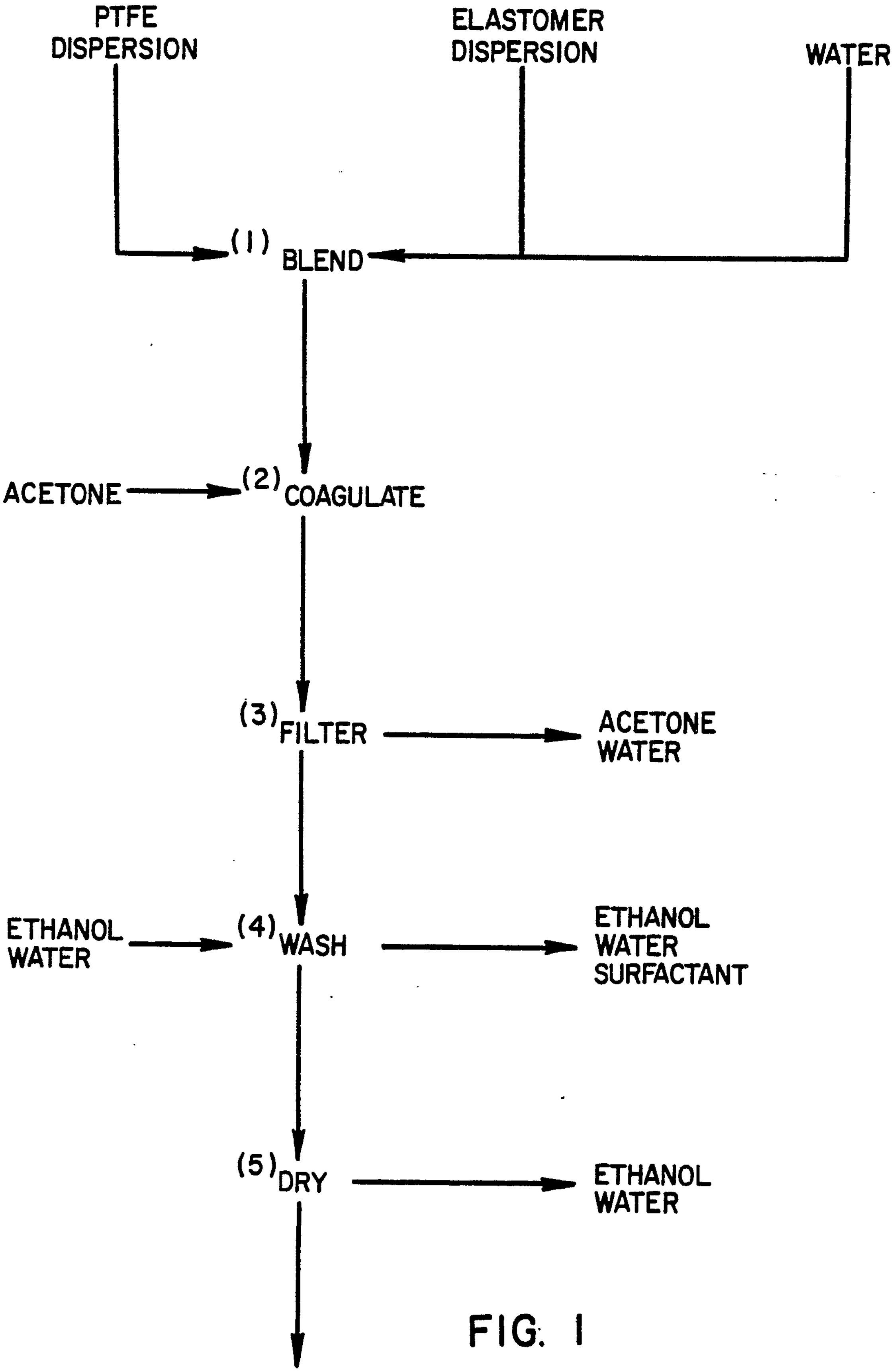
PTFE DISPERSION
ELASTOMER DISPERSION
WATER
(1) BLEND
ACETONE
(2) COAGULATE
(3) FILTER
ACETONE WATER
ETHANOL WATER
(4) WASH
ETHANOL WATER SURFACTANT
(5) DRY
ETHANOL WATER

FIG. 1

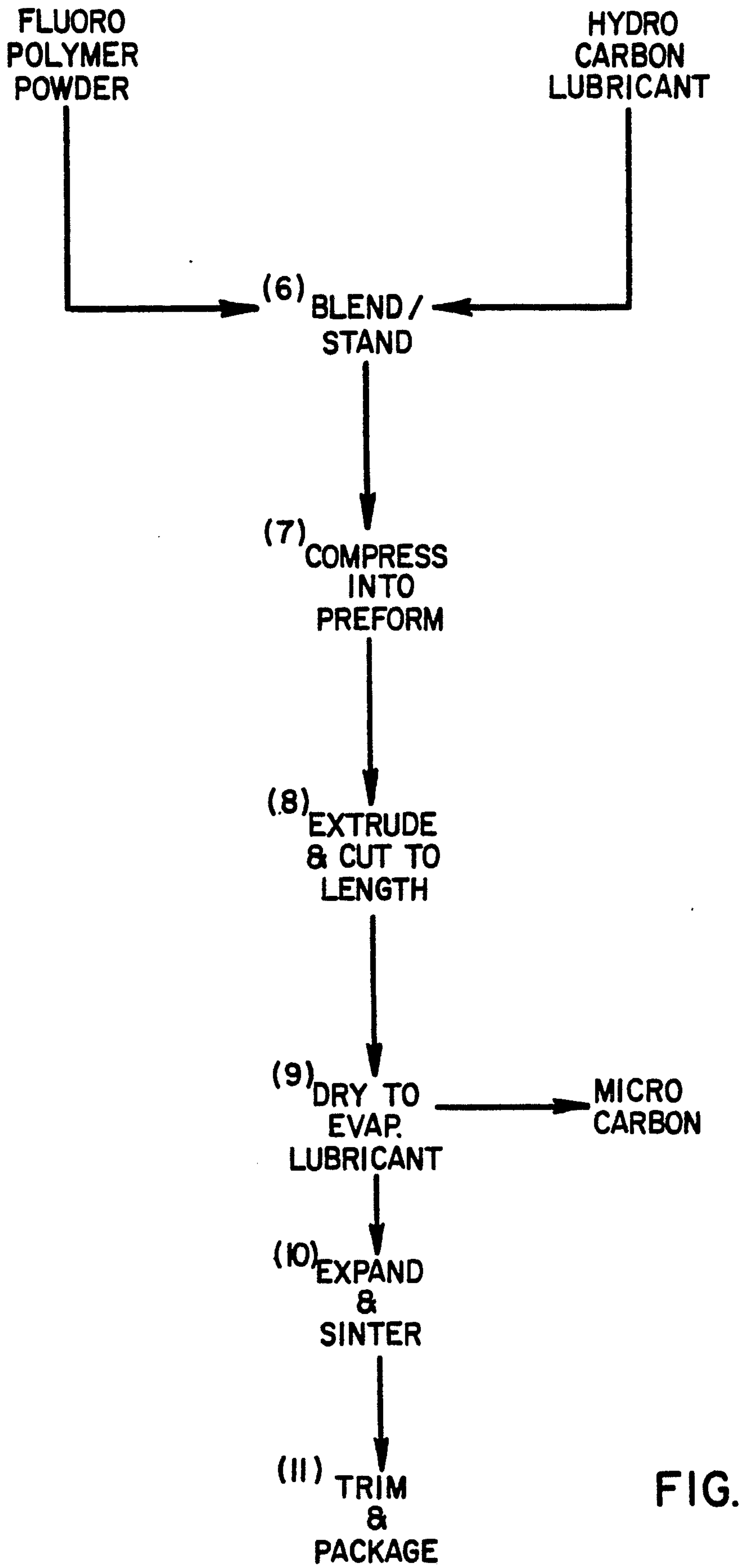

FLUORO POLYMER POWDER
HYDRO CARBON LUBRICANT
(6) BLEND / STAND
(7) COMPRESS INTO PREFORM
(8) EXTRUDE & CUT TO LENGTH
(9) DRY TO EVAP. LUBRICANT
MICRO CARBON
(10) EXPAND & SINTER
(11) TRIM & PACKAGE


FIG. 2

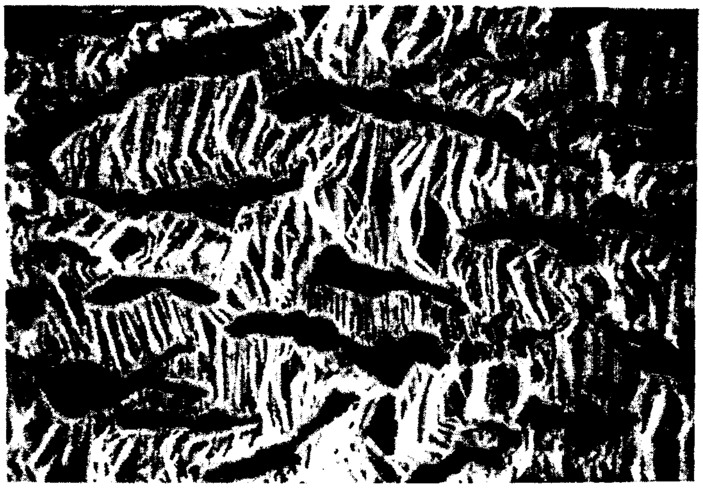

FIG. 3

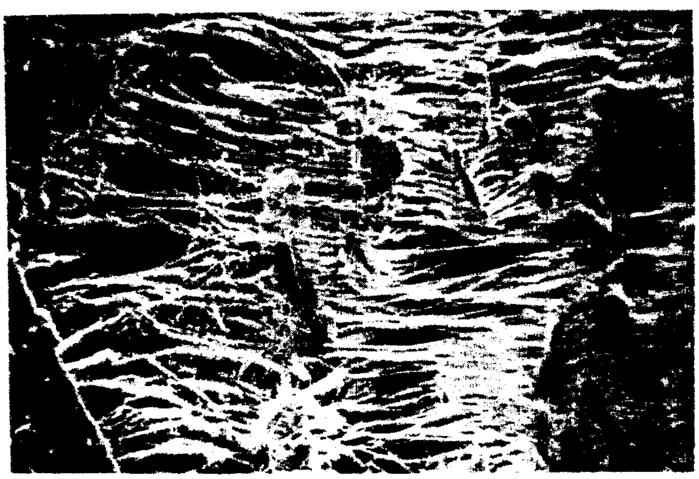

FIG. 3a

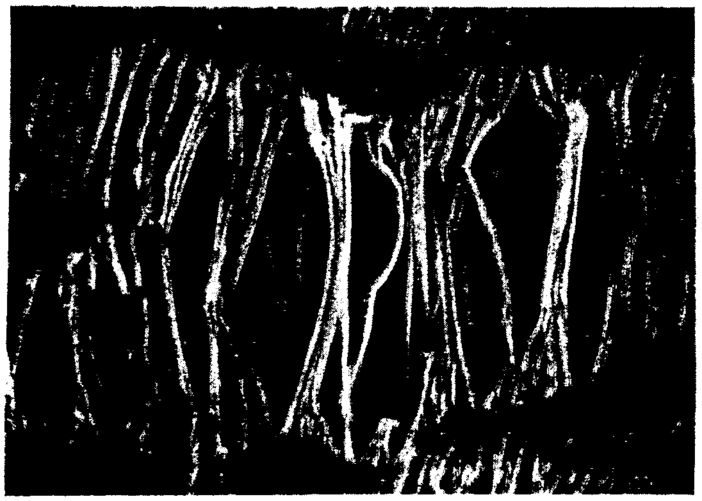

FIG. 3b

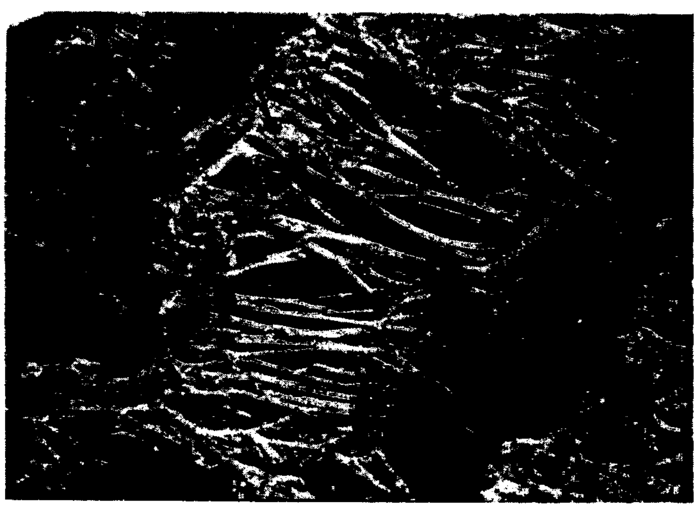

FIG. 3c

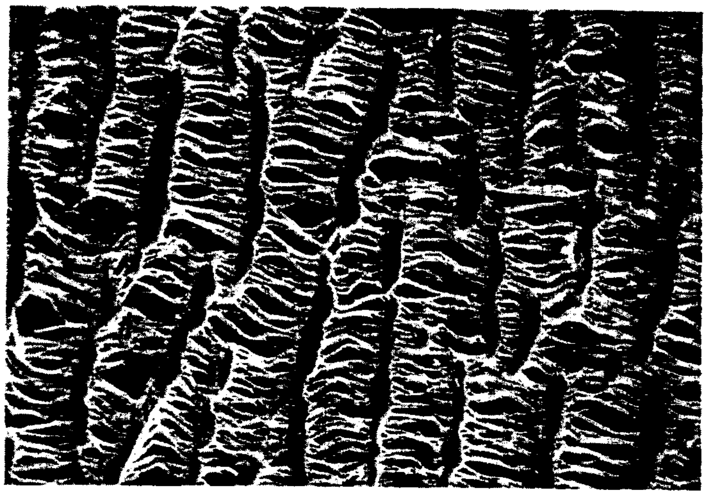

FIG. 3d

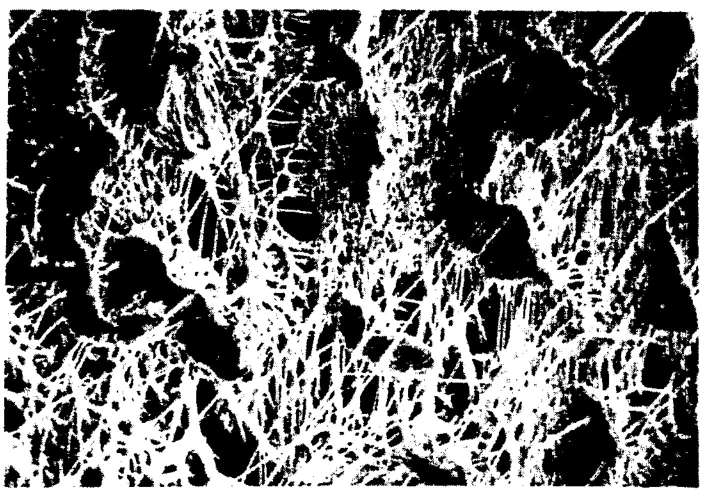

FIG. 3e

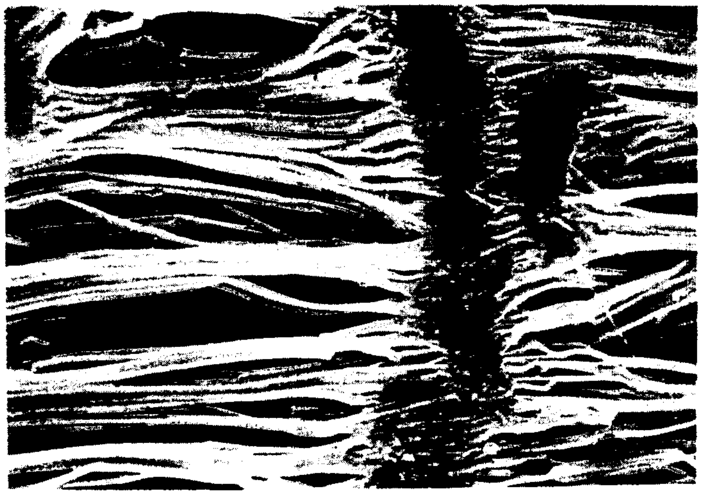

FIG. 4

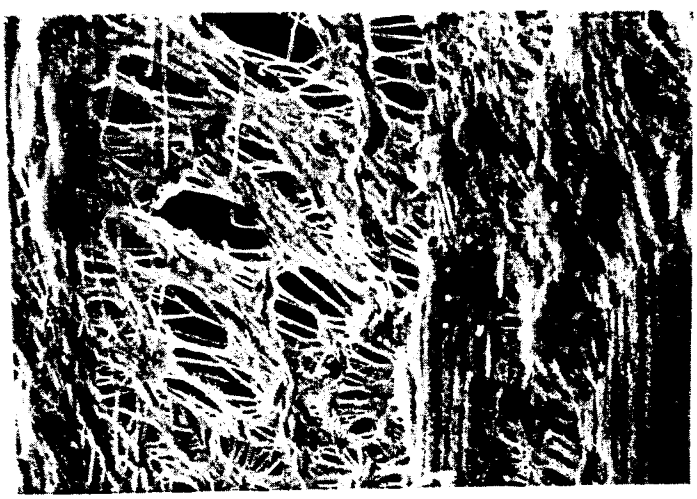

FIG. 4a

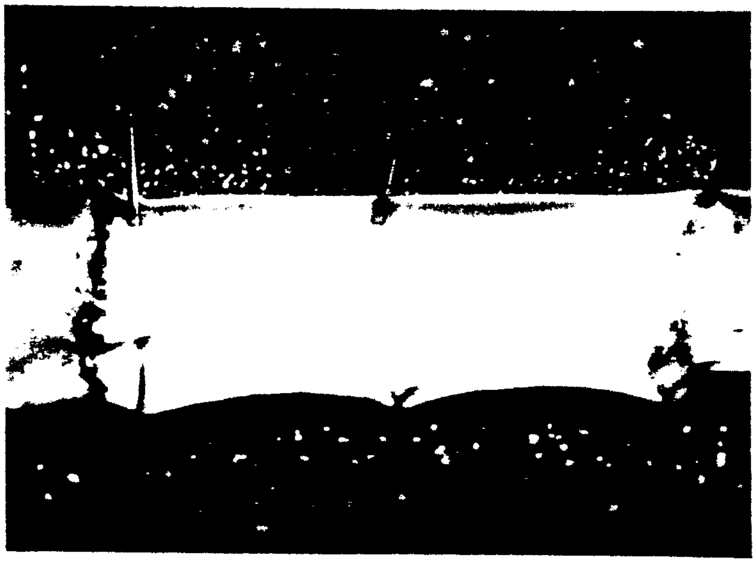

FIG. 5

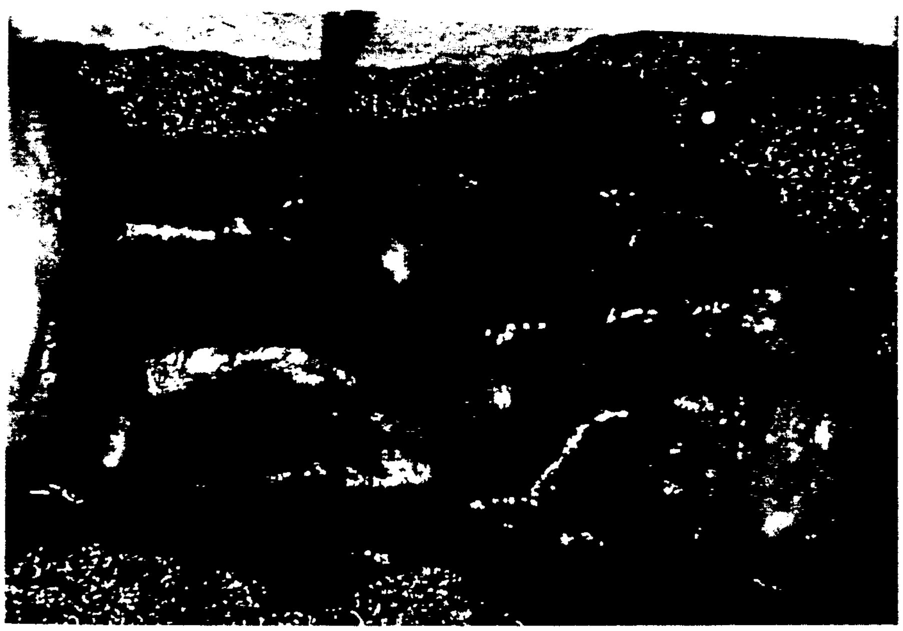

FIG. 6

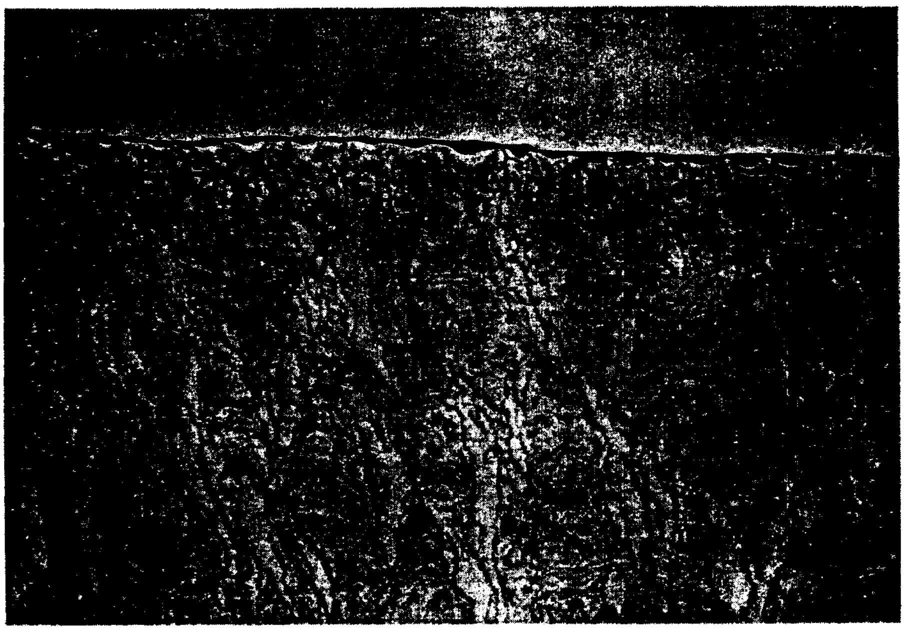

FIG. 6a

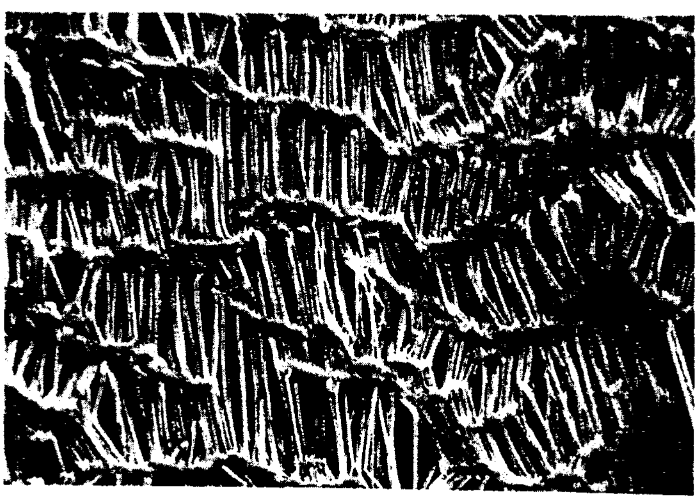

FIG. 7

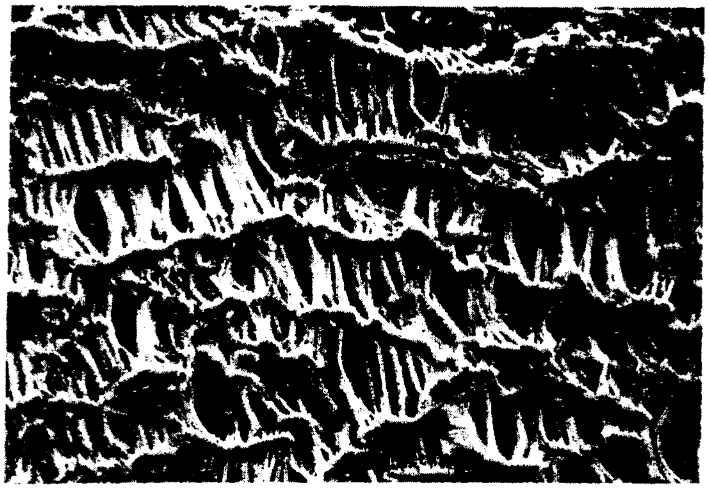

FIG. 7a

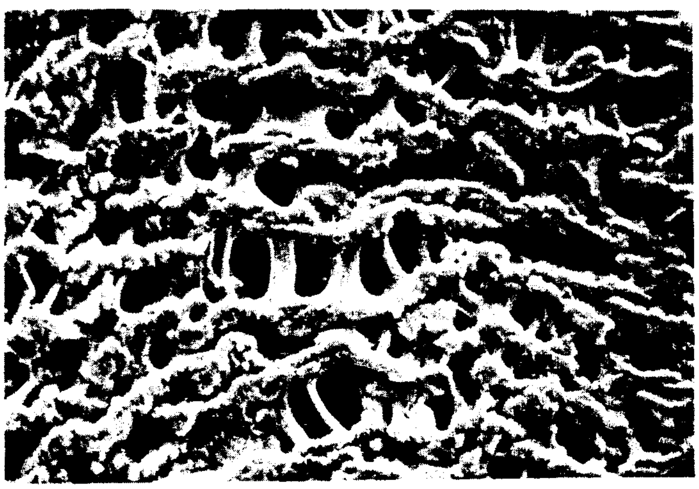

FIG. 7b

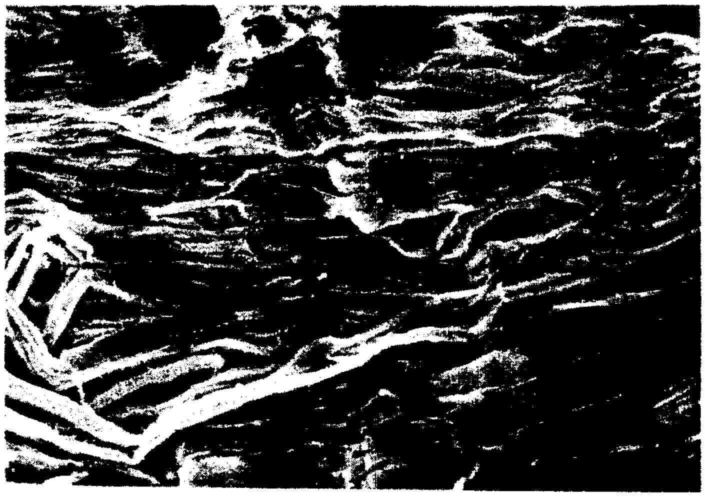

FIG. 8

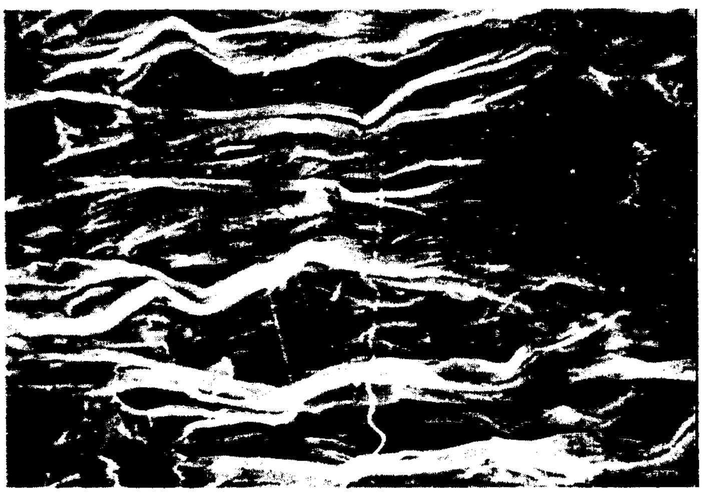

FIG. 8a

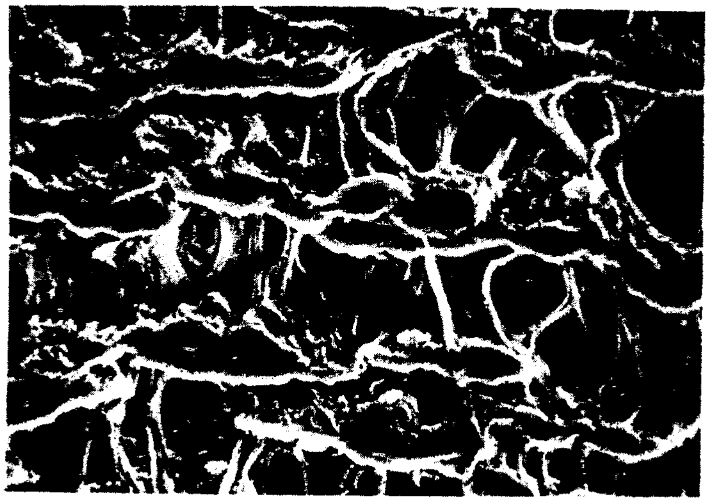

FIG. 8b

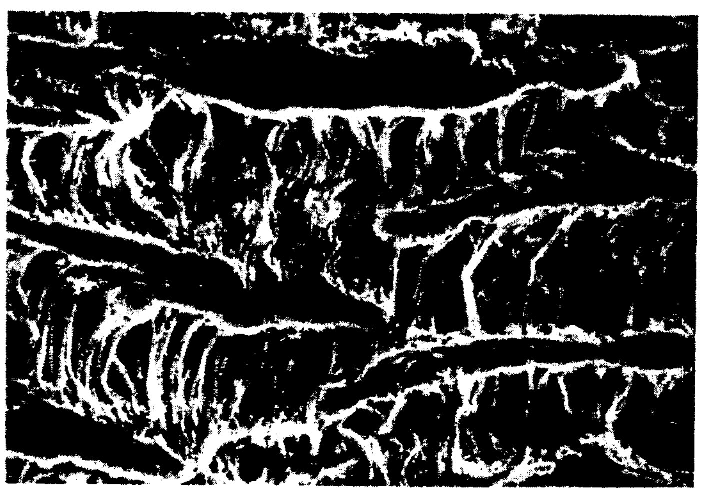

FIG. 8c

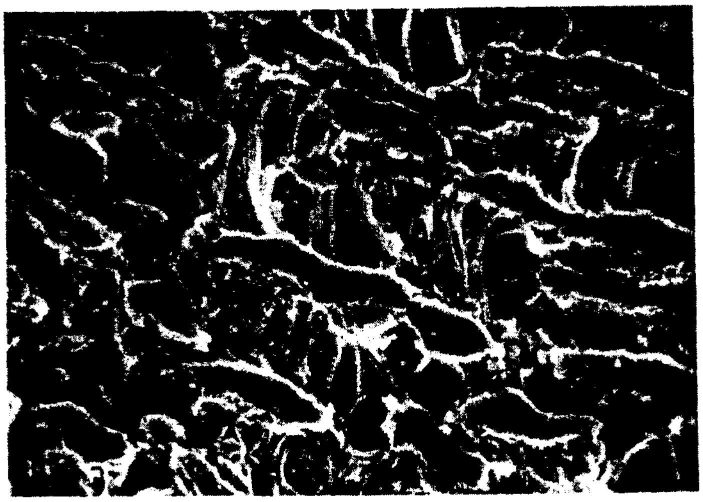

FIG. 8d

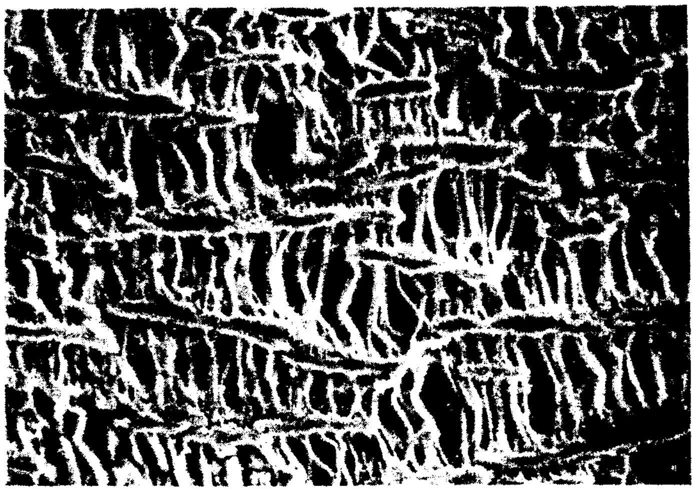

FIG. 9a

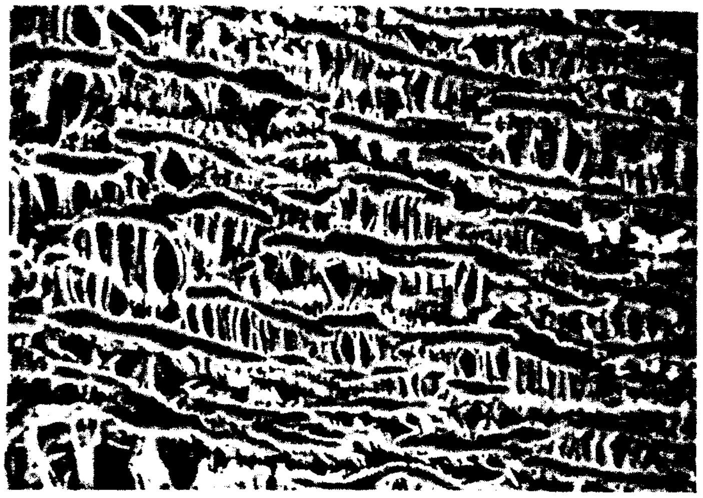

FIG. 9b

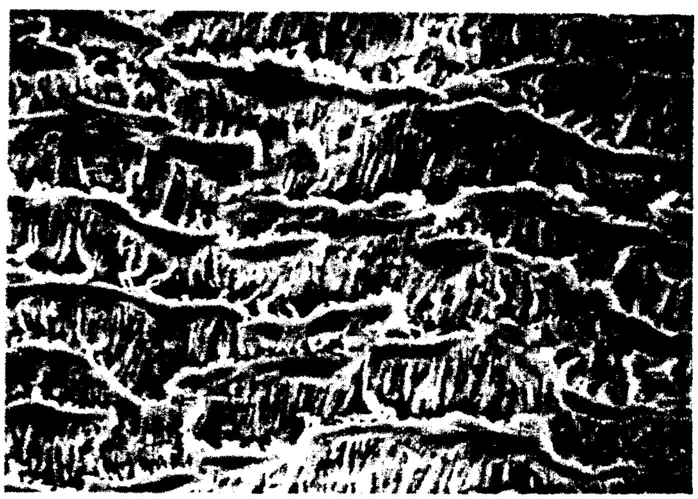

FIG. 9c